# EUROPÄISCHE PATENTSCHRIFT

(11) **EP 1 180 395 B1**
(45) Veröffentlichungstag und Bekanntmachung des Hinweises auf die Patenterteilung: **05.03.2008**
(21) Anmeldenummer: 01119563.3
(22) Anmeldetag: 15.08.2001
(51) Int. Cl.: B01J 19/24, B01J 19/00, B01J 8/02

(54) **Reaktor zur Durchführung einer stark wärmegetönten katalytischen Reaktion**
Reactor for carrying out a strongly thermic reaction
Réacteur pour la mise en oeuvre d'une réaction fortement thermique

(30) Priorität: 17.08.2000 DE 10040209
(43) Veröffentlichungstag der Anmeldung: 20.02.2002
(73) Patentinhaber: Linde Aktiengesellschaft, 80807 München (DE)
(72) Erfinder: Schödel, Nicole, Dr. Dipl.-Chem., 81477 München (DE); Sotzek, Manfred, Dipl.-Phys., 81477 München (DE); Süssmann, Wolfgang, Dipl.-Ing., 81479 München (DE); Walzl, Roland, Dipl.-Ing., 82340 Feldafing (DE)
(74) Vertreter: Zahn, Christoph

(56) Entgegenhaltungen:
- EP-A- 0 830 893
- EP-A- 0 885 653
- US-A- 3 528 783
- US-A- 5 162 288
- US-A- 5 538 700
- US-A- 5 846 494
- US-A- 6 118 038

## Beschreibung

Die Erfindung betrifft einen Reaktor zur Durchführung einer stark wärmegetönten katalytischen Reaktion in einem Prozeßfluid, ausgerüstet mit in einem Abstand parallel zueinander angeordneten Platten, die mit einander gegenüberliegenden seitlichen Begrenzungsflächen flache Kanäle bilden, wobei ein Teil der Kanäle einen festen Katalysator enthält und das Prozeßfluid leitet und ein anderer Teil der Kanäle ein Wärmeübertragungsmedium in indirektem Wärmekontakt mit dem Prozeßfluid führt.

Katalytische Prozesse sind häufig mit hohen Energieumsätzen verbunden. Meist muss ein bestimmter Temperaturbereich eingehalten werden, um einen hohen Umsatz und eine hohe Ausbeute an erwünschten Produkten (Selektivität) zu erzielen und eine Schädigung des verwendeten Katalysators zu vermeiden. Adiabate Reaktoren mit Zwischenkühlung führen zu einer Vielzahl von Komponenten oder zu einem konstruktiv aufwendigen Reaktor. Bekannt sind auch Festbettreaktoren mit Kühl- und Heizeinrichtungen im Bett. Meist handelt es sich um Rohrbündelreaktoren mit einem Festbett und mit Wärmeträger-führenden Rohren im Festbett, wie sie in allen Standardwerken über Reaktionstechnik, beispielsweise in Ullmann's Encyclopedia of Industrial Chemistry, VCH 1992, Vol. 4B beschrieben sind. Ein Reaktor mit gewickelten Kühlmittelrohren im Festbett ist in M. Lehmbeck "Linde Isothermal Reactor for Methanol Synthesis" 41 (1986) Seiten 5 bis 8 veröffentlicht:

Aus der Zeitschrift Hydrocarbon Processing, March 1997, Seite 134 ist ein Röhrenreaktor mit Katalysatorpartikeln in den Rohren bekannt. Die Rohre werden auf der Mantelseite des Reaktors mit siedendem Wasser oder anderen geeigneten Wärmeträgern gekühlt. Die Aufteilung des Reaktionsraumes und der Katalysatorpartikel auf mehrere Rohre stellt sicher, dass im Falle einer Betriebsstörung eine sich selbst beschleunigende Reaktion, hervorgerufen durch örtliche Überhitzung, sich auf ein Reaktionsrohr beschränkt und nicht den ganzen Reaktor erfasst. Diese Reaktorkonstruktion hat sich bewährt, weist jedoch auch mehrere Nachteile auf.
- Der Reaktormantel muss auf den in der Praxis oft hohen Kühlmitteldruck ausgelegt sein. Dadurch ist der Mantel sehr dick und damit teuer und der Reaktor schwer zu transportieren.
- Die Rohrböden sind bei großem Durchmesser ebenfalls sehr dick und durch Wärmespannungen gefährdet.
- Die vielen Reaktionsrohre sind nur mit großem Aufwand zu befüllen. Insbesondere ist auf gleichmäßige Befüllung mit gleichem Druckverlust in den verschiedenen Rohren zu achten, damit nicht ein wegen hohem Druckabfall zu wenig beaufschlagtes Reaktionsrohr überhitzt wird.
- Wegen des hohen Gewichts wird für den Reaktor meist C-Stahl verwendet, obwohl Rost damit unvermeidlich ist. Rost wirkt aber für viele Reaktionen als ein Katalysatorgift.

Aus der DE 198 04 806 A1 ist ein Plattenwärmetauscher mit Katalysatorfestbett bekannt. Er enthält gekühlte Trennwände in der Schüttung. Der Reaktormantel muss nur für den Druck des Reaktionsgases ausgelegt werden, der Reaktor benötigt keine Rohrböden. Er ist somit insgesamt leichter als ein Röhrenreaktor und deshalb mit geringeren Kosten auch aus Edelstahl zu fertigen.

Der der Erfindung am nächsten liegenden Stand der Technik ist in der Patentschrift US 3 528 783 offenbart. Mit gebogenen Blechen gebildete Rechteckkanäle leiten teils ein Reaktionsmedium und teils einen Wärmeträger. Die Kanäle mit dem Reaktionsmedium enthalten festes Katalysatormaterial Sandwich-artig zwischen Kanälen mit dem Wärmeträger.

Diesem katalytischen Vielschichtreaktor - wie auch anderen Reaktoren mit Katalysatorschüttung - haftet der Nachteil an, dass sich im Betrieb in den das Katalysatormaterial enthaltenden Kanälen quer zur Strömungsrichtung des Reaktionsmediums ein Temperaturprofil ausbildet. Dadurch kann nur eine mittlere Temperatur eingestellt werden. Ein optimaler Umsatz und eine optimale Selektivität kann deshalb prinzipiell nicht erreicht werden. Außerdem können eine örtliche Überhitzung des Katalysatormaterials und ein Ausufern der Reaktion in einen unerwünschten oder gar gefährlichen Bereich nicht ausgeschlossen werden. Der genannte Vielschichtreaktor hat außerdem den Nachteil, dass für ihn ein druckfester Außenbehälter benötigt wird.

Aufgabe der Erfindung ist es daher, diese Nachteile zu vermeiden.

Diese Aufgabe wird erfindungsgemäß gelöst von einem Reaktor mit den Merkmalen des Anpruchs 1. Ausführungen der Erfindung sind Gegenstand von Unteransprüchen.

Kennzeichnend an der Erfindung ist, dass die Platten eben sind oder mit Rillen oder Rippen versehen sind und auf der dem Prozeßfluid zugewandten Oberfläche mindestens teilsweise mit dem Katalysator beschichtet sind. Mit dem erfindungsgemäßen Reaktor kann sich ein nennenswertes Temperaturprofil quer zur Strömungsrichtung nicht ausbilden, da die Wärmezufuhr oder -abfuhr auf kürzestem Wege nämlich über die Platten und die auf den Platten aufgetragene Schicht an das Wärmeträgermedium erfolgt. Noch leichter als bei Kanälen (oder Rohren) mit Katalysatorschüttung wird außerdem eine gleichmäßige Durchströmung aller Kanäle erreicht. Mit nur teilweiser Beschichtung können in Teilen des Reaktors Wärmeübertragungszonen ohne katalytische Reaktion ausgebildet werden.

Bei dem Reaktor werden die seitlichen Begrenzungsflächen als Mantelstücke ausgebildet die mit den Platten und Sammlern für das Prozeßfluid und für das Wärmeübertragungsmedium einen druckfesten quaderförmigen Block mit Kanälen bilden. Der Vorteil besteht darin, dass der Reaktor sowohl auf der Prozeßfluidseite als auch auf der Wärmeträgerseite mit Betriebsdrücken von mehr als 25 bar beaufschlagt sein kann.

Die das Prozeßfluid leitenden Kanäle können gewellte oder gefaltete Bleche (fins) enthalten, die Durchgänge für das Prozeßfluid ausbilden. Der Wärmeübergang zwischen den Prozeßfluid und dem Wärmeträger wird durch die fins verbessert.

Die fins können gelocht sein und so eine Strömungsverbindung zwischen den Durchgängen ausbilden.

Die fins können beidseitig mindestens teilweise mit Katalysatormaterial beschichtet sein. Bei gleicher Dicke der Beschichtung wird auf diese Art pro Reaktorvolumen mehr wirksame Katalysatoroberfläche installiert.

Die Katalysatorschicht kann ein Trägermaterial enthalten.

Die Katalysatorschicht kann eine Schichtdicke zwischen 1 und 500 µm, bevorzugt zwischen 10 und 100 µm, besitzen.

Der erfindungsgemäße Reaktor kann aus Aluminium oder aus Stahl oder Edelstahl gefertigt sein.

Eine besonders vorteilhafte Verwendung erfährt der erfindungsgemäße Reaktor, wenn im Reaktor eine endotherme oder eine exotherme Reaktion durchgeführt wird. Ohne die Verwendbarkeit des Reaktors auf die nun folgenden Beispiele zu beschränken, kommen z. B. folgende Verwendungen in Betracht.

Der erfindungsgemäße Reaktor kann mit Vorteil verwendet werden bei der
- Methanolsynthese,
- Synthese höherer Alkohole,
- Hydrierung von Kohlenwasserstoffen,
- selektiven Hydrierung von C₂H₂ zu C₂H₄,
- nicht-selektiven Hydrierung von C₂H₄ zu C₂H₆,
- Methanisierung oder Methansynthese,
- Kohlenmonoxid-Konvertierung,
- Fischer-Tropsch-Synthese
- Epoxidierung,
- Synthese von Ethylenoxid,
- Claus-Reaktion,
- Direktoxidation von H₂S zu Schwefel,
- Oxidation von SO₂ zu SO₃
- oder bei der NH₃-Synthese.

### Beispiel

Bei einer selektiven Hydrierung von Acetylen zu Ethylen mit einem erfindungsgemäßen Reaktor mit fins und den Betriebsdaten

| | |
|---|---|
| Druck | 31 bar |
| Temperatur | 70-80 °C |
| Durchsatz | 170 000 kg/h |

und unter Einsatz von flüssigem Butan zur Kühlung wird ein Reaktor mit 13 m³ Volumen

| | |
|---|---|
| Länge | 6 m |
| Breite | 1,2 m |
| Tiefe | 1,8 m |
| Gewicht ohne Katalysator | 16 t |

benötigt, der in den Katalysator-beschichteten Kanälen einen Druckabfall von etwa 150 mbar besitzt. Ein vergleichbarer Reaktor nach dem Stand der Technik besitzt ein um den Faktor 4 bis 10 größeres Volumen.

Die Erfindung wird anhand einer Ausführungsform mit einer Figur näher erläutert.

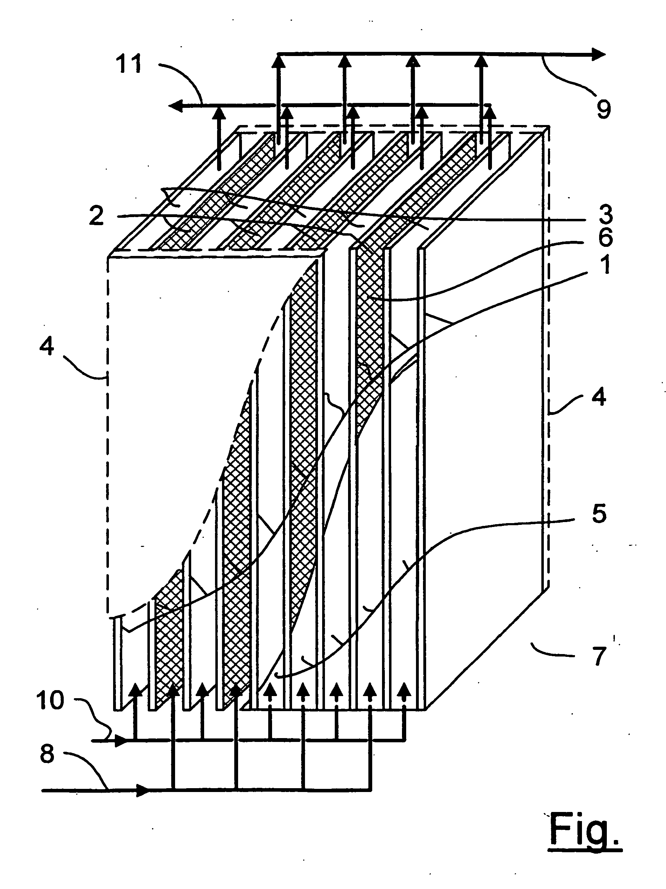

Die Figur zeigt einen erfindungsgemäßen Reaktor in dreidimensionaler Darstellung.

Schematisch dargestellt ist in der Figur der prinzipielle Aufbau eines solchen Reaktors. Die Funktion des Reaktors wird anhand des Beispiels der selektiven Hydrierung von Acetylen zu Ethylen beschrieben.

In einem Abstand parallel zueinander angeordnete Platten 1 bilden mit einander gegenüberliegenden seitlichen Begrenzungsflächen Kanäle 2 für ein Prozeßfluid und Kanäle 3 für ein Kühlmedium. Die Begrenzungsflächen können als Platten 4 (in der Figur gestrichelt dargestellt) oder als Stege 5 zwischen den Platten 1 und/oder (in der Figur nicht dargestellt) zwischen fins und den Platten 1 ausgeführt sein. Die Plattenoberflächen im Inneren der das Prozeßfluid führenden Kanäle sind mit

Katalysatormaterial 6 beschichtet. Nicht in der Figur dargestellt sind Sammler für das Prozeßfluid und das Kühlmedium, die zusammen mit den Platten einen formstabilen und druckfesten Reaktor 7 bilden.

Dem Reaktor 7 wird beispielsweise ein Acetylen-haltiger Strom 8 zugeführt. In den Kanälen 2 wird an dem Katalysatormaterial 6 das Acetylen zu Ethylen hydriert und ein Strom 9 mit dem Ethylen gewonnen. Die am Katalysatormaterial gebildete Prozeßwärme wird über die Platten an flüssiges Butan abgeführt, das mit dem Strom 10 den Kanälen 3 zugeführt wird, bei der Wärmeaufnahme dort verdampft und als gasförmiger Strom 11 abgeführt wird.

Durch die Wärmeabfuhr direkt am Entstehungsort werden Nebenreaktionen wie die Bildung von Ethan oder von Oligomeren (z. B. Anthracenöl oder Grünöl) weitgehend vermieden und bei einem sicheren Betrieb des erfindungsgemäßen Reaktors eine hohe Ethylenausbeute erzielt.

## Patentansprüche

1. Reaktor zur Durchführung einer stark wärmegetönten katalytischen Reaktion in einem Prozessfluid, ausgerüstet mit in einem Abstand parallel zueinander angeordneten Platten, die mit einander gegenüberliegenden seitlichen Begrenzungsflächen flache Kanäle bilden, wobei ein Teil der Kanäle einen festen Katalysator enthält und das Prozessfluid leitet und ein anderer Teil der Kanäle ein Wärmeübertragungsmedium in indirektem Wärmekontakt mit dem Prozessfluid führt, wobei die Platten eben oder mit Rillen oder Rippen versehen und auf der dem Prozessfluid zugewandten Oberfläche mindestens teilweise mit dem Katalysator beschichtet sind, **dadurch gekennzeichnet, dass** die seitlichen Begrenzungsflächen als Mantelstücke ausgebildet sind, die mit den Platten und Sammlern für das Prozessfluid und für das Wärmeübertragungsmedium einen druckfesten quaderförmigen Block mit Kanälen bildender sowohl auf Seiten des Prozessfluids als auch auf Seiten des Wärmeträgermediums mit Betriebsdrücken von mehr als 25bar beaufschlagbar ist.

2. Reaktor nach Anspruch 1, **dadurch gekennzeichnet, dass** die das Prozessfluid leitenden Kanäle gewellte oder gefaltete Bleche (fins) enthalten, die Durchgänge für das Prozessfluid ausbilden.

3. Reaktor nach Anspruch 2, **dadurch gekennzeichnet, dass** die fins gelocht sind und damit eine Strömungsverbindung zwischen den Durchgängen ausbilden.

4. Reaktor nach Anspruch 2 oder 3, **dadurch gekennzeichnet, dass** die fins beidseitig mindestens teilweise mit Katalysatormaterial beschichtet sind.

5. Reaktor nach einem der Ansprüche 1 bis 4, **dadurch gekennzeichnet, dass** die Katalysatorschicht ein Trägermaterial enthält.

6. Reaktor nach einem der Ansprüche 1 bis 5, **dadurch gekennzeichnet, dass** die Katalysatorschicht eine Schichtdicke zwischen 1 und 500 µm besitzt.

7. Reaktor nach einem der Ansprüche 1 bis 6, **dadurch gekennzeichnet, dass** die Katalysatorschicht eine Schichtdicke zwischen 10 und 100 µm besitzt.

8. Reaktor nach einem der Ansprüche 1 bis 7, **dadurch gekennzeichnet, dass** der Reaktor aus Aluminium gefertigt ist.

9. Reaktor nach einem der Ansprüche 1 bis 8, **dadurch gekennzeichnet, dass** der Reaktor aus Stahl oder Edelstahl gefertigt ist.

10. Verwendung des Reaktors nach einem der Ansprüche 1 bis 9, **dadurch gekennzeichnet, dass** im Reaktor eine endotherme oder eine exotherme Reaktion durchgeführt wird.

11. Verwendung des Reaktors nach Anspruch 10 bei der Methanolsynthese oder der Synthese höherer Alkohole.

12. Verwendung des Reaktors nach Anspruch 10 bei der Hydrierung von Kohlenwasserstoffen.

13. Verwendung des Reaktors nach Anspruch 12 bei der selektiven Hydrierung von C₂H₂ zu C₂H₄.

14. Verwendung des Reaktors nach Anspruch 13 bei der nicht-selektiven Hydrierung von C₂H₄ zu C₂H₆.

15. Verwendung des Reaktors nach Anspruch 10 bei der Methanisierung oder bei der Methansynthese.

16. Verwendung des Reaktors nach Anspruch 10 bei der Kohlenmonoxid-Konvertierung.

17. Verwendung des Reaktors nach Anspruch 10 bei der Fischer-Tropsch-Synthese.

18. Verwendung des Reaktors nach Anspruch 10 bei der Epoxidierung.

19. Verwendung des Reaktors nach Anspruch 18 bei der Synthese von Ethylenoxid.

20. Verwendung des Reaktors nach Anspruch 10 bei der Claus-Reaktion.

21. Verwendung des Reaktors nach Anspruch 10 bei der Direktoxidation von H₂S zu Schwefel.

22. Verwendung des Reaktors nach Anspruch 10 bei der Oxidation von SO₂ zu SO₃.

23. Verwendung des Reaktors nach Anspruch 10 bei der NH₃-Synthese.

## Claims

1. Reactor for carrying out a catalytic reaction having a high heat of reaction in a process fluid, equipped with plates which are arranged parallel to one another with a spacing between them and together with lateral faces located opposite one another form flat channels, with part of the channels containing a solid catalyst and conducting the process fluid and another part of the channels conducting a heat transfer medium in indirect heat contact with the process fluid and the plates being flat or provided with grooves or ribs and being coated on at least part of the surface facing the process fluid with the catalyst, **characterized in that** the lateral faces are configured as casing pieces which together with the plates and collectors for the process fluid and for the heat transfer medium form a pressure-resistant cuboidal channel-containing block which can be subjected to operating pressures of more than 25 bar both on the side of the process fluid and on the side of the heat transfer medium.

2. Reactor according to Claim 1, **characterized in that** the channels conducting the process fluid contain corrugated or folded metal sheets (fins) which form passages for the process fluid.

3. Reactor according to Claim 2, **characterized in that** the fins are perforated and thus form a flow connection between the passages.

4. Reactor according to Claim 2 or 3, **characterized in that** the fins are at least partly coated with catalyst on both sides.

5. Reactor according to any of Claims 1 to 4,
**characterized in that** the catalyst layer contains a support material.

6. Reactor according to any of Claims 1 to 5,
**characterized in that** the catalyst layer has a thickness in the range from 1 to 500 µm.

7. Reactor according to any of Claims 1 to 6,
**characterized in that** the catalyst layer has a thickness in the range from 10 to 100 µm.

8. Reactor according to any of Claims 1 to 7,
**characterized in that** the reactor is made of aluminium.

9. Reactor according to any of Claims 1 to 8,
**characterized in that** the reactor is made of steel or stainless steel.

10. Use of the reactor according to any of Claims 1 to 9, **characterized in that** an endothermic reaction or an exothermic reaction is carried out in the reactor.

11. Use of the reactor according to Claim 10 in the synthesis of methanol or the synthesis of higher alcohols.

12. Use of the reactor according to Claim 10 in the hydrogenation of hydrocarbons.

13. Use of the reactor according to Claim 12 in the selective hydrogenation of C₂H₂ to C₂H₄.

14. Use of the reactor according to Claim 13 in the nonselective hydrogenation of C₂H₄ to C₂H₆.

15. Use of the reactor according to Claim 10 in methanation or in the synthesis of methane.

16. Use of the reactor according to Claim 10 in the converting of carbon monoxide.

17. Use of the reactor according to Claim 10 in the Fischer-Tropsch synthesis.

18. Use of the reactor according to Claim 10 in epoxidation.

19. Use of the reactor according to Claim 18 in the synthesis of ethylene oxide.

20. Use of the reactor according to Claim 10 in the Claus reaction.

21. Use of the reactor according to Claim 10 in the direct oxidation of H₂S to sulphur.

22. Use of the reactor according to Claim 10 in the oxidation of SO₂ to SO₃.

23. Use of the reactor according to Claim 10 in the synthesis of NH₃.

## Revendications

1. Réacteur destiné à réaliser une réaction catalytique fortement endo/exothermique dans un fluide de traitement, équipé de plaques disposées parallèlement les unes aux autres à intervalles les unes des autres et qui forment des canaux plats avec des surfaces frontières latérales mutuellement opposées, une partie des canaux contenant un catalyseur solide et conduisant le fluide de traitement et une autre partie des canaux amenant un fluide caloporteur en contact thermique indirect avec le fluide de traitement, les plaques étant planes ou dotées de rainures ou de nervures et étant recouvertes au moins en partie du catalyseur sur leur surface tournée vers le fluide de traitement,
**caractérisé en ce que**
les surfaces latérales de délimitation sont configurées comme morceaux d'enveloppe qui forment avec les plaques, les collecteurs de fluide de traitement et les collecteurs de fluide caloporteur un bloc parallélépipédique résistant à la pression, doté de canaux et sur lequel des pressions de travail supérieures à 25 bars peuvent être appliquées tant du côté du fluide de traitement que du côté du fluide caloporteur.

2. Réacteur selon la revendication 1, **caractérisé en ce que** les canaux qui conduisent le fluide de traitement contiennent des tôles ondulées ou pliées (ailettes) qui forment des passages pour le fluide de traitement.

3. Réacteur selon la revendication 2, **caractérisé en ce que** les ailettes sont perforées et forment ainsi une communication d'écoulement entre les passages.

4. Réacteur selon les revendications 2 ou 3,
**caractérisé en ce qu'**au moins une partie des ailettes est recouverte des deux côtés de matériau de catalyseur.

5. Réacteur selon l'une des revendications 1 à 4, **caractérisé en ce que** la couche de catalyseur contient un matériau de support.

6. Réacteur selon l'une des revendications 1 à 5, **caractérisé en ce que** la couche de catalyseur a une épaisseur comprise entre 1 et 500 µm.

7. Réacteur selon l'une des revendications 1 à 6, **caractérisé en ce que** la couche de catalyseur a une épaisseur comprise entre 10 et 100 µm.

8. Réacteur selon l'une des revendications 1 à 7, **caractérisé en ce que** le réacteur est réalisé en aluminium.

9. Réacteur selon l'une des revendications 1 à 8, **caractérisé en ce que** le réacteur est réalisé en acier ou en acier allié.

10. Utilisation du réacteur selon l'une des revendications 1 à 9, **caractérisée en ce qu'**une réaction endothermique ou exothermique est réalisée dans le réacteur.

11. Utilisation du réacteur selon la revendication 10 en synthèse de méthanol ou en synthèse d'alcools supérieurs.

12. Utilisation du réacteur selon la revendication 10 en hydrogénation d'hydrocarbures.

13. Utilisation du réacteur selon la revendication 12 pour l'hydrogénation sélective de C₂H₂ en C₂H₄.

14. Utilisation du réacteur selon la revendication 13 en hydrogénation non sélective de C₂H₄ en C₂H₆.

15. Utilisation du réacteur selon la revendication 10 en méthanisation ou synthèse de méthane.

16. Utilisation du réacteur selon la revendication 10 en conversion de monoxyde de carbone.

17. Utilisation du réacteur selon la revendication 10 en synthèse de Fischer-Tropsch.

18. Utilisation du réacteur selon la revendication 1.0 en époxydation.

19. Utilisation du réacteur selon la revendication 18 en synthèse d'oxyde d'éthylène.

20. Utilisation du réacteur selon la revendication 10 pour la réaction de Claus.

21. Utilisation du réacteur selon la revendication 10 en oxydation directe de H₂S en soufre.

22. Utilisation du réacteur selon la revendication 10 en oxydation de SO₂ en SO₃.

23. Utilisation du réacteur selon la revendication 10 en synthèse de NH₃.
